# EUROPEAN PATENT SPECIFICATION

(11) **EP 0 503 604 B1**
(45) Date of publication and mention of the grant of the patent: **27.12.1996**
(21) Application number: 92104201.6
(22) Date of filing: 11.03.1992
(51) Int. Cl.: C07D 205/095, C07D 205/09, C07D 499/87, C07D 499/86

(54) **Allenyl beta-lactam compounds and process for preparing same**
Allenyl-Beta-Lactam-Verbindungen und Verfahren zu deren Herstellung
Dérivés d'allényl-bêta-lactame et leur procédé de préparation

(30) Priority: 11.03.1991 JP 72448/91
(43) Date of publication of application: 16.09.1992
(73) Proprietor: OTSUKA KAGAKU KABUSHIKI KAISHA, Osaka-shi Osaka-fu 540 (JP)
(72) Inventor: Torii, Sigeru, Akaiwa-gun, Okayama-ken (JP); Tanaka, Hideo, Okayama-shi, Okayama-ken (JP); Taniguchi, Masatoshi, c/o Otsuka Kagaku K.K., Osaka-shi, Osaka-fu (JP); Sasaoka, Michio, c/o Otsuka Kagaku K.K., Tokushima-shi, Tokushima-ken (JP); Shiroi, Takashi, c/o Otsuka Kagaku K.K., Tokushima-shi, Tokushima-ken (JP); Kameyama, Yutaka, c/o Otsuka Kagaku K.K., Tokushima-shi, Tokushima-ken (JP)
(74) Representative: Barz, Peter, Dr.

(56) References cited:
- EP-A- 0 114 729
- EP-A- 0 445 822
- FR-A- 2 392 027
- FR-A- 2 555 578
- CHEMICAL ABSTRACTS, vol. 116, 1992, Columbus, Ohio, US; abstract no. 128438e,

## Description

An object of the present invention is to provide novel allenyl β-lactam compounds which are useful as intermediates, for example, in the synthesis of antibiotics of the penam, penem or cephem type having a broad antibacterial spectrum. A further object is to provide a process for preparing these allenyl β-lactam compounds.

The present invention relates to novel allenyl β-lactam compounds represented by the formula (1) 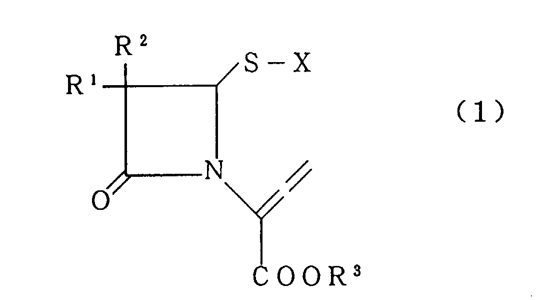 wherein R¹ is a hydrogen atom, halogen atom, amino or protected amino group, R² is a hydrogen atom, halogen atom, lower C₁₋₄ alkoxyl, lower C₁₋₄ acyl, lower C₁₋₄ alkyl substituted with hydroxyl or protected hydroxyl, hydroxyl or protected hydroxyl, R¹ and R² representing =O when taken together, R³ is a hydrogen atom or carboxylic acid protecting group, and X is the group -SO₂R⁴ or the group -SR⁴, R⁴ being substituted or unsubstituted aryl or a substituted or unsubstituted nitrogen-containing aromatic heterocyclic group, the substituent(s) of R⁴ being selected from halogen, hydroxyl, nitro, cyano, aryl, lower C₁₋₄ alkyl, amino, mono lower C₁₋₄ alkylamino, di lower C₁₋₄ alkylamino, mercapto, alkylthio or arylthio represented by the group R⁶S- (wherein R⁶ is lower C₁₋₄ alkyl or aryl), formyloxy, acyloxy represented by the group R⁶COO-, formyl, acyl represented by the group R⁶CO-, alkoxyl or aryloxy represented by the group R⁶O-, carboxyl, alkoxycarbonyl or aryloxycarbonyl represented by the group R⁶OCO-.

The compounds of the invention represented by formula (1) are novel compounds which have not been disclosed in the literature and which can be prepared, for example, by reacting an azetidinone derivative represented by the formula (2) with a base. 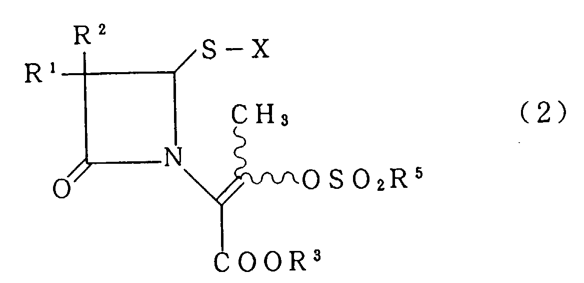 wherein R¹, R², R³ and X are as defined above, and R⁵ is substituted or unsubstituted lower C₁₋₄ alkyl or substituted or unsubstituted aryl, the substituent(s) being defined as for R⁴ above.

Examples of groups mentioned herein are as follows. The term "halogen atom" as used hereinafter means, for example, fluorine, chlorine, bromine or iodine atoms, unless otherwise specified. The term "lower alkyl" means a straight-chain or branched C₁₋₄ alkyl group such as methyl, ethyl, n-propyl, isopropyl, n-butyl, isobutyl, sec-butyl or tert-butyl. The term "aryl" means, for example, phenyl, naphthyl or the like.

Exemplary of the protected amino represented by R¹ are phenoxyacetamido, p-methylphenoxyacetamido, p-methoxyphenoxyacetamido, p-chlorophenoxyacetamido, p-bromophenoxyacetamido, phenylacetamido, p-methylphenylacetamido, p-methoxyphenylacetamido, p-chlorophenylacetamido, p-bromophenylacetamido, phenylmonochloroacetamido, phenyldichloroacetamido, phenylhydroxyacetamido, phenylacetoxyacetamido, α - oxophenylacetamido, thienylacetamido, benzamido, p-methylbenzamido, p-tert-butylbenzamido, p-methoxybenzamido, p-chlorobenzamido and p-bromobenzamido groups, the groups disclosed in Theodora W. Greene, "Protective Groups in Organic Synthesis" (hereinafter referred to merely as the "literature"), Chap. 7 (pp. 218∼ 287), phenylglycylamido group, phenylglycylamido groups having protected amino, p-hydroxyphenylglycylamido group, and p-hydroxyphenylglycylamido groups having protected amino and/or protected hydroxyl. Examples of protective groups for amino are those disclosed in the literature, Chap. 7 (pp. 218∼ 287). Examples of protective groups for the hydroxyl of p-hydroxyphenylglycylamido group are those disclosed in the literature, Chap.2 (pp. 10∼ 72).

The lower alkoxyl groups represented by R² are straight-chain or branched C_{1∼4} alkoxyl groups such as methoxy, ethoxy, n-propoxy, isopropoxy, n-butoxy, isobutoxy, sec-butoxy and tert-butoxy groups.

The lower acyl groups represented by R² are straight-chain or branched C_{1∼4} acyl groups such as formyl, acetyl, propionyl, butyryl and isobutyryl.

Examples of protective groups for the protected hydroxyl in the lower alkyl represented by R² and substituted with hydroxyl or protected hydroxyl, and for the protected hydroxyl represented by R² are those disclosed in the literature, Chap. 2 (pp. 10~72). The substituted lower alkyl represented by R² may have as its substituent(s) one or at least two same or different groups selected from among hydroxyl and the protected hydroxyl groups. Such substituent(s) may be positioned on at least one carbon atom of the alkyl.

Exemplary of the carboxylic acid protecting group represented by R³ are benzyl, p-methoxybenzyl, p-nitrobenzyl, diphenylmethyl, trichloroethyl, tert-butyl, and those disclosed in the literature, Chap. 5 (pp. 152~192).

While R⁴ represents a nitrogen-containing aromatic heterocyclic group which may have a substituent or substituents, exemplary of the nitrogen-containing aromatic hetrocyclic group are thiazol-2-yl, thiadiazol-2-yl, benzothiazol-2-yl, oxazol-2-yl, benzoxazol-2-yl, imidazol-2-yl, benzoimidazol-2-yl, pyrimidinyl, pyridyl and the like.

The substituents which may be substituted in the aryl or nitrogen-containing aromatic heterocyclic group represented by R⁴ are selected from halogen atoms, hydroxyl, nitro, cyano, aryl, lower alkyl, amino, mono lower alkylamino, di lower alkylamino, mercapto, alkylthio or arylthio represented by the group R⁶S- (wherein R⁶ is lower alkyl or aryl), formyloxy, acyloxy represented by the group R⁶COO- (wherein R⁶ is as defined above), formyl, acyl represented by the group R⁶CO- (wherein R⁶ is as defined above), alkoxyl or aryloxy represented by R⁶O- (wherein R⁶ is as defined above), carboxyl, alkoxycarbonyl or aryloxycarbonyl represented by the group R⁶OCO- (wherein R⁶ is as defined above). The aryl or nitrogen-containing aromatic heterocyclic group represented by R⁴ may have one or at least two same or different groups selected from among the above substituents.

R⁵ represents lower alkyl or aryl which may have a substituent or substituents, which are those mentioned for R⁴. The lower alkyl or aryl represented by R⁵ may have one or at least two same or different groups selected from among the foregoing substituents. Such substituent(s) may be positioned on at least one carbon atom of the alkyl or aryl.

The azetidinone derivative of the formula (2) for use as the starting material in the invention is prepared, for example, by the process disclosed in JP-A-165367/1986.

To prepare the compound of the formula (1) according to the invention, the compound of the formula (2) is reacted with a base in a suitable solvent. The base to be used is preferably an aliphatic or aromatic amine. Examples of such amines are triethylamine, diisopropylamine, ethyldiisopropylamine, tributylamine, DBN(1,5-diazabicyclo[3.4.0]nonene-5), DBU(1,8-diazabicyclo[5.4.0]undecene-7), DABCO(1,4-diazabicyclo[2.2.2]octane), piperidine, N-methylpiperidine, 2,2,6,6-tetramethylpiperidine, morpholine, N-methylmorpholine, N,N-dimethylaniline, N,N-dimethylaminopyridine and the like.

The base is used usually in an amount of 1 to 12 moles, preferably 1 to 6 moles, per mole of the compound of the formula (2). The solvent to be used can be any of a wide variety of those which dissolve the compound of the formula (2) and which are inert under the reaction condition employed. Examples of useful solvents are lower alkyl esters of lower carboxylic acids such as methyl formate, ethyl formate, propyl formate, butyl formate, methyl acetate, ethyl acetate, propyl acetate, butyl acetate, methyl propionate and ethyl propionate; ethers such as diethyl ether, ethyl propyl ether, ethyl butyl ether, dipropyl ether, diisopropyl ether, dibutyl ether, methyl cellosolve and dimethoxyethane; cyclic ethers sush as tetrahydrofuran and dioxane; nitriles such as acetonitrile, propionitrile, butyronitrile, isobutyronitrile and valeronitrile; substituted or unsubstituted aromatic hydrocarbons such as benzene, toluene, xylene, chlorobenzene and anisole; hydrocarbon halides such as dichloromethane, chloroform, dichloroethane, trichloroethane, dibromoethane, propylene dichloride, carbon tetrachloride and Freons; hydrocarbons such as pentane, hexane, heptane and octane; cycloalkanes such as cyclopentane, cyclohexane, cycloheptane and cyclooctane; amides such as dimethylformamide and dimethylacetamide; dimethyl sulfoxide; etc. These solvents are used singly or in admixture. The solvent may contain water. The solvent is used preferably in an amount of about 0.5 to about 200 liters, more preferably about 1 to about 50 liters, per kg of the compound of the formula (2). The reaction is conducted at -70 °C to 100 °C, preferably -50 °C to 50 °C. The compound of the invention can be obtained as a substantially pure product by subjecting the resulting reaction mixture to extraction or crystallization in the usual manner, while the product can of course be purified by other method.

The present invention provides allenyl β -lactam compounds which are useful as intermediates for synthesizing antibiotics of the penam, penem or cephem type; cf. copending European applications EP-A-503 597, 503 603 and 507 124.

The invention will be described in greater detail with reference to the following examples. Ph stands for phenyl.

### Example 1

Compound of the formula (3) → compound of formula (4)

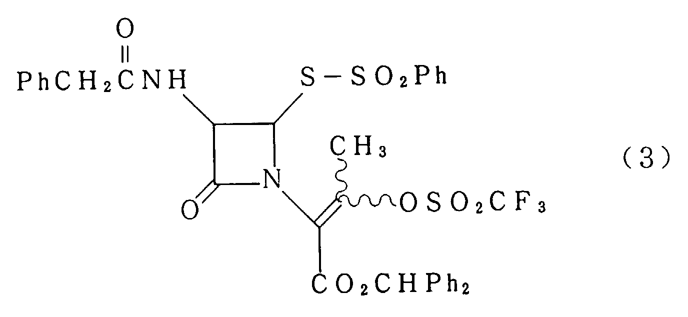

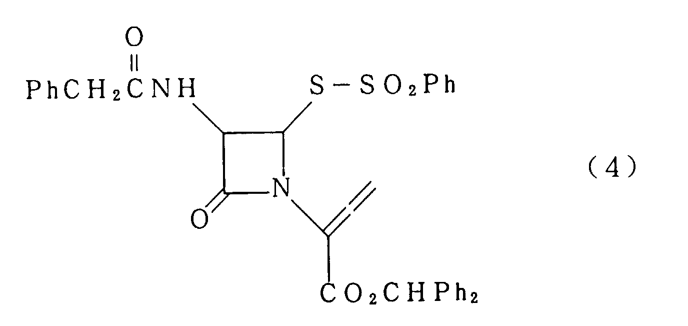

One gram of the compound of the formula (3) (R¹ = phenylacetamido, R² = H, R³ = diphenylmethyl, X = phenylsulfonyl, R⁵ = trifluoromethyl) was dissolved in 10 ml of N,N-dimethylformamide. After cooling the solution to -30 °C, 0.43 ml of triethylamine was added to the solution, followed by stirring at -30 °C for 1 hour for reaction. The reaction mixture was extracted with ethyl acetate, and the extract was washed with water and then dried over anhydrous sodium sulfate. Concentration of the dried extract gave the compound of the formula (4) (R¹, R², R³ and X are the same as above) in a yield of 99 %.
NMR (CDCl₃); δ ppm
3.61 (s, 2H), 5.31 (dd, 1H, J=5Hz and 7Hz), 5.57 and 5.70 (ABq, 2H, J=15Hz), 5.84 (d, 1H, J=5Hz), 6.02 (d, 1H, J=7Hz), 6.81 (s, 1H), 7.22∼ 7.73 (m, 20H)

### Examples 2 to 7

The following compounds (4) to (8) were each prepared from starting compound listed in Table 1 by the same reaction as in Example 1.

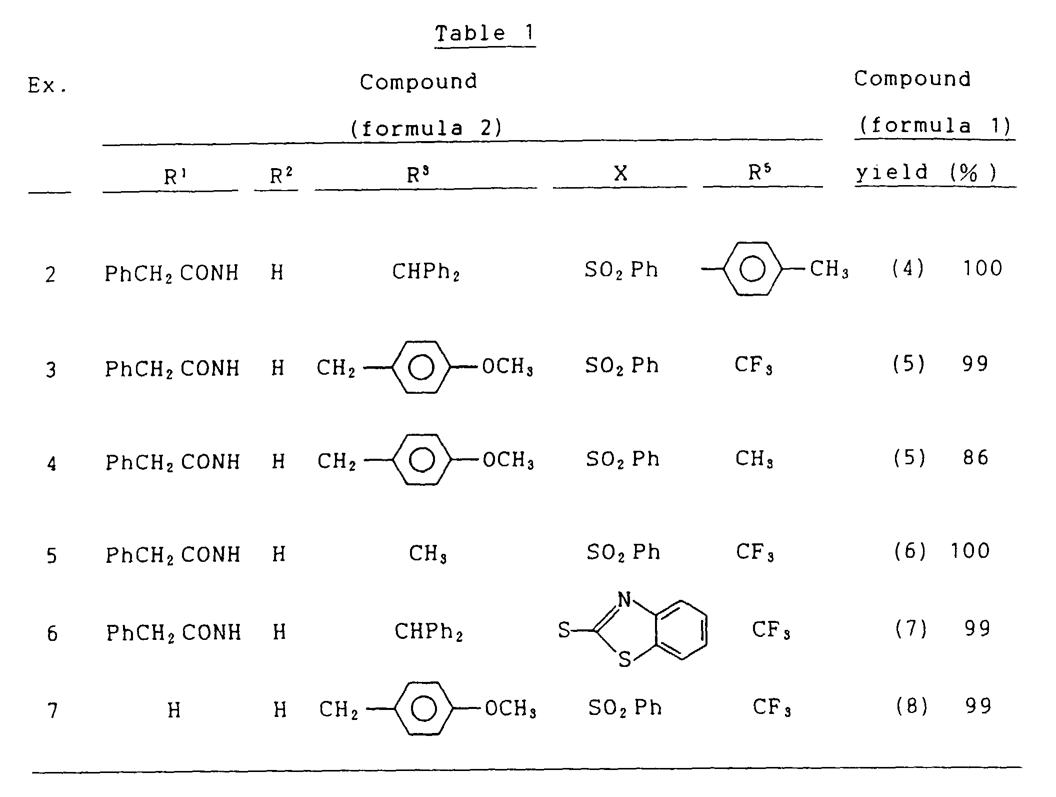

NMR data as to the compounds (5) to (8) is collectively given below.
- Compound (5) :: 3.58 (s, 2H), 3.80 (s, 3H), 5.10 (s, 2H), 5.32 (dd, 1H, J=5Hz and 8Hz), 5.60 and 5.47 (ABq, 2H, J=15Hz) , 5.87 (d, 1H, J=5Hz), 6.08 (d, 1H, J=8Hz), 6.85∼ 7.83 (m, 14Hz)
- Compound (6) :: 3.59 (s, 2H), 3.74 (s, 3H), 5.33 (dd, 1H, J=5Hz and 8Hz), 5.54 and 5.64 (ABq, 2H, J=15Hz), 5.88 (d, 1H, J=5Hz), 6.02 (d, 1H, 8Hz), 7.20∼ 7.90 (m, 10Hz)
- Compound (7) :: 3.67 (s, 2H), 5.25 (dd, 1H, J=5Hz and 8Hz), 5.69 (d, 1H, J=5Hz), 5.60 and 5.76 (ABq, 2H, J=15Hz), 6.71 (s, 1H), 7.00∼ 7.34 (m, 20Hz)
- Compound (8) ;: 3.02 (dd, 1H, J=2.6Hz and 15.7 Hz), 3.58 (dd, 1H, J=5.4Hz and 15.7Hz), 3.79 (s, 3H), 5.17 (s, 2H), 5.47 and 5.60 (ABq, 2H, J=15.2Hz), 5.62 (dd, 1H, J=2.6Hz and 5.4Hz), 6.87∼ 7.89 (m, 9H)

### Examples 8 to 10

The compound of the formula (4) was obtained in the yield shown in Table 2 by the same reaction as in Example 1 with the exception of changing the solvent and temperature for the reaction as listed in Table 2.

**Table 2**

| Ex. | solvent | temperature | yield (% ) |
|---|---|---|---|
| 8 | tetrahydrofuran | -20 °C | 99 |
| 9 | tetrahydrofuran | 20 °C | 98 |
| 10 | methylene chloride | -20 °C | 95 |

### Reference Example 1

Compound of formula (5) → compound of formula (9)

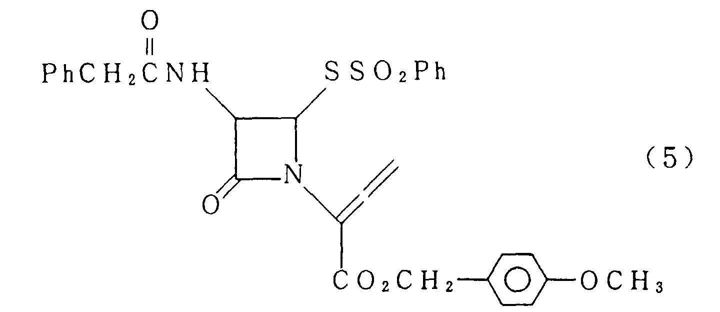

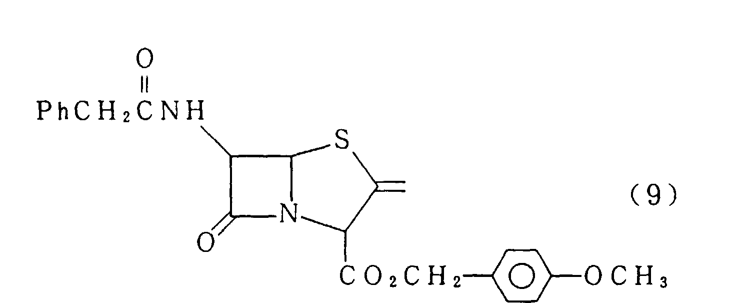

A 100 mg quantity of the compound of the formula (5) (R¹ = phenylacetamido, R² = H, R³ = p methoxybenzyl, X = phenylsulfonyl) was dissolved in 1 ml of N,N-dimethylformamide, followed by the addition of 50 mg of zinc powder and then 50 mg of BiCl₃ to the solution. The mixture was reacted at room temperature for 30 minutes with stirring. To the reaction mixture thus obtained was added 1N hydrochloric acid, followed by extraction with ethyl acetate. The resulting organic layer was separated off, washed with water, dried over anhydrous magnesium sulfate and concentrated in a vacuum. The residue obtained was purified by silica gel column chromatography, giving the compound of the formula (9) (R¹ = phenylacetamido, R² = H, R³ = p-methoxybenzyl) in a yield of 92 % .
- NMR (CDCl₃) ;: δ ppm
3.61 (ABq, 2H, J=16Hz), 3.80 (s, 3H), 5.11 (s, 2H), 5.18 (t, 1H, J=1Hz), 5.24 (t, 1H, J=1Hz), 5.35 (t, 1H, J=1Hz), 5.57 (d, 1H, J=4Hz), 5.75 (dd, 1H, J=4Hz and 9Hz), 6.07 (d, 1H, J=9Hz), 6.85~7.40 (m, 9H)

### Reference Example 2

Compound of formula (4) → compound of formula (10)

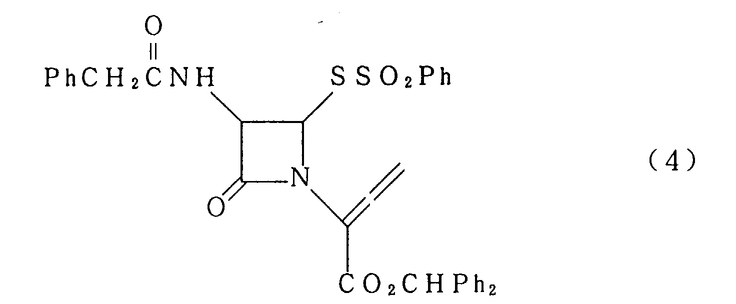

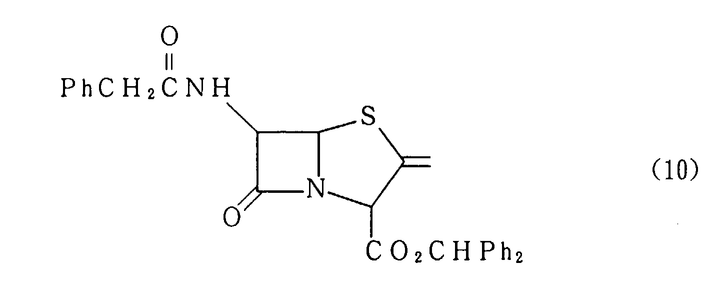

The same reaction as in Reference Example 1 was conducted using 200 mg of the compound of the formula (4) (R¹ = phenylacetamido, R² = H, R³ = diphenylmethyl, X = phenylsulfonyl) as the starting material to obtain the compound of the formula (10) (R¹ = phenylacetamido, R² = H, R³ = diphenylmethyl) in a yield of 89 %.
- NMR (CDCl₃) ;: δ ppm
3.62 (s, 2H), 5.26∼ 5.28 (m, 2H), 5.37 (t, 1H, J=2Hz), 5.61 (d, 1H, J=4Hz), 5.76 (dd, 1H, J=4Hz and 9Hz), 6.14 (d, 1H, J=9Hz), 6.82 (s, 1H), 7.20∼ 7.41 (m, 15H)

### Reference Example 3

Compound of formula (6) → compound of formula (11)

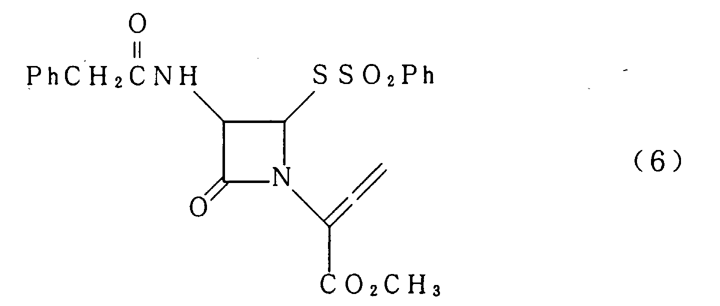

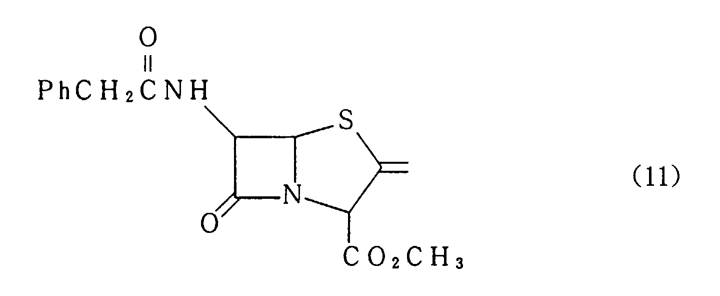

A 500 mg quantity of the compound of the formula (6) (R¹ = phenylacetamido, R² = H, R³ = methyl, X = phenylsulfonyl) was dissolved in 0.5 ml of N,N-dimethylformamide. To the solution were added 50 mg of zinc powder and 10 µl of TiCl₄, and the mixture was stirred at room temperature for 25 minutes for reaction. The reaction mixture was thereafter treated in the same manner as in Reference Example 1, giving the compound of the formula (11) (R¹ = phenylacetamido, R² = H, R³ = methyl) in a yield of 95 %.
- NMR (CDCl₃) ;: δ ppm
3.62 (ABq, 2H, J=16Hz), 3.78 (s, 3H), 5.19 (t, 1H, J=2Hz), 5.28 (t, 1H, J=2Hz), 5.40 (t, 1H, J=2Hz), 5.60 (d, 1H, J=4Hz), 5.77 (dd, 1H, J=4Hz and 9Hz), 6.20 (d, 1H, J=9Hz), 7.27~7.39 (m, 5H)

### Reference Example 4

Compound of formula (8) → compound of formula (12)

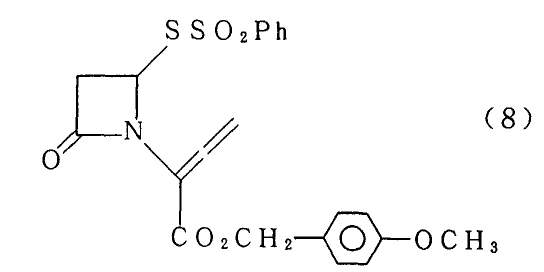

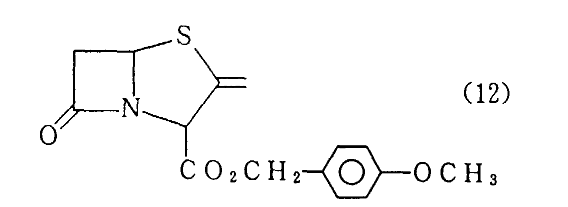

The same reaction as in Reference Example 1 was conducted using 189 mg of the compound of the formula (8) (R¹ = R² = H, = = p-methoxybenzyl, X = phenylsulfonyl) as the starting material to obtain the compound of the formula (12) (R¹ = R² = H, R³ = p-methoxybenzyl) in a yield of 88 %.
- NMR (CDCl₃) ;: δ ppm
3.16 (dd, 1H, J=1.5Hz and 16Hz), 3.66 (dd, 1H, J=4Hz and 16Hz), 3.82 (s, 3H), 5.13 (s, 2H), 5.24 (dd, 1H, J=1.8Hz and 1.8Hz), 5.28 (dd, 1H, J=1.8Hz and 1.8Hz), 5.32 (dd, 1H, J=1.8Hz and 1.8Hz), 5.38 (dd, 1H, J=1.5Hz and 4Hz), 6.87∼ 7.30 (m, 4H)

## Claims

1. An allenyl β-lactam compound represented by the formula (1) 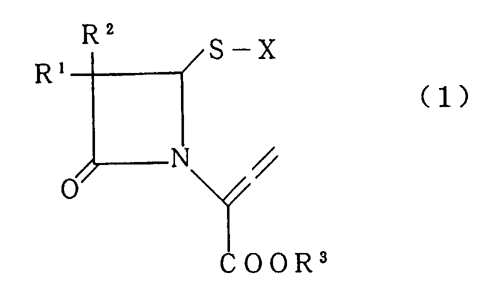 wherein R¹ is a hydrogen atom, halogen atom, amino or protected amino group, R² is a hydrogen atom, halogen atom, lower C₁₋₄ alkoxyl, lower C₁₋₄ acyl, lower C₁₋₄ alkyl substituted with hydroxyl or protected hydroxyl, hydroxyl or protected hydroxyl, R¹ and R² representing =O when taken together, R³ is a hydrogen atom or carboxylic acid protecting group, and X is the group -SO₂R⁴ or the group -SR⁴, R⁴ being substituted or unsubstituted aryl or a substituted or unsubstituted nitrogen-containing aromatic heterocyclic group, the substituent(s) of R⁴ being selected from halogen, hydroxyl, nitro, cyano, aryl, lower C₁₋₄ alkyl, amino, mono lower C₁₋₄ alkylamino, di lower C₁₋₄ alkylamino, mercapto, alkylthio or arylthio represented by the group R⁶S- (wherein R⁶ is lower C₁₋₄ alkyl or aryl), formyloxy, acyloxy represented by the group R⁶COO-, formyl, acyl represented by the group R⁶CO-, alkoxyl or aryloxy represented by the group R⁶O-, carboxyl, alkoxycarbonyl or aryloxycarbonyl represented by the group R⁶OCO-.

2. A process for preparing an allenyl β-lactam compound of formula (1) according to claim 1 characterized in that an azetidinone derivative represented by the formula (2) 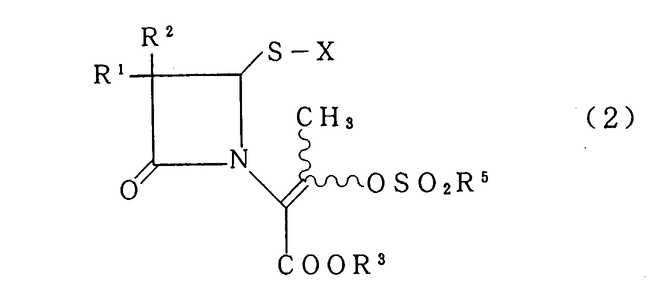 wherein R¹, R², R³ and X are as defined in claim 1, and R⁵ is substituted or unsubstituted lower C₁₋₄ alkyl or substituted or unsubstituted aryl, the substituent(s) being defined as for R⁴ in claim 1,
is reacted with a base.

3. The use of an allenyl β-lactam compound of formula (1) according to claim 1 as an intermediate in the synthesis of an antibiotic of the penam, penem or cephem type.

## Patentansprüche

1. Allenyl-β-lactam-Verbindung, dargestellt durch die Formel (1) 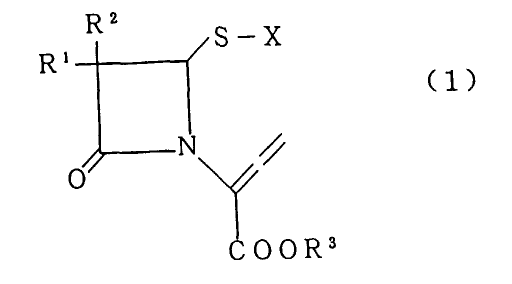 worin R¹ ein Wasserstoffatom, ein Halogenatom, eine Amino- oder geschützte Aminogruppe ist, R² für ein Wasserstoffatom, ein Halogenatom, Nieder-C₁₋₄-alkoxyl, Nieder-C₁₋₄-acyl, Nieder-C₁₋₄-alkyl, das mit Hydroxyl oder geschütztem Hydroxyl substituiert ist, Hydroxyl oder geschütztes Hydroxyl steht, wobei R¹ und R² für =O stehen, wenn sie zusammengenommen werden, R³ ein Wasserstoffatom oder eine Carbonsäure-Schutzgruppe bedeutet und X die Gruppe -SO₂R⁴ oder die Gruppe -SR⁴ darstellt, wobei R⁴ substituiertes oder unsubstituiertes Aryl oder eine substituierte oder unsubstituierte stickstoffhaltige aromatische heterocyclische Gruppe repräsentiert, wobei der oder die Substituent(en) von R⁴ ausgewählt ist bzw. sind aus Halogen, Hydroxyl, Nitro, Cyano, Aryl, Nieder-C₁₋₄-alkyl, Amino, Mono-nieder-C₁₋₄-alkylamino, Di-nieder-C₁₋₄-alkylamino, Mercapto, durch die Gruppe R⁶S-dargestelltem Alkylthio oder Arylthio (worin R⁶ Nieder-C₁₋₄-alkyl oder Aryl bedeutet), Formyloxy, durch die Gruppe R⁶COO- dargestelltem Acyloxy, Formyl, durch die Gruppe R⁶CO- dargestelltem Acyl, durch die Gruppe R⁶O-dargestelltem Alkoxyl oder Aryloxy, Carboxyl, durch die Gruppe R⁶OCO- dargestelltem Alkoxycarbonyl oder Aryloxycarbonyl.

2. Verfahren zur Herstellung einer Allenyl-β-lactam-Verbindung der Formel (1) gemäß Anspruch 1, dadurch gekennzeichnet, daß ein durch die Formel (2) dargestelltes Azetidinon-Derivat 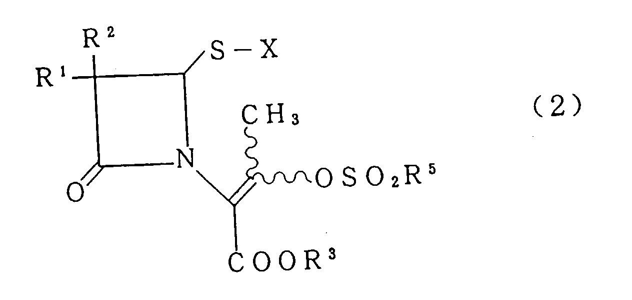 worin R¹, R², R³ und X wie in Anspruch 1 definiert sind und R⁵ für substituiertes oder unsubstituiertes Nieder-C₁₋₄-alkyl oder substituiertes oder unsubstituiertes Aryl steht, wobei der bzw. die Substituent(en) wie für R⁴ in Anspruch 1 definiert ist bzw. sind,
mit einer Base umgesetzt wird.

3. Verwendung einer Allenyl-β-lactam-Verbindung der Formel (1) nach Anspruch 1 als Zwischenprodukt in der Synthese eines Antibiotikums des Penam-, Penem- oder Cephem-Typs.

## Revendications

1. Un allényl-β-lactame représenté par la formule (1) 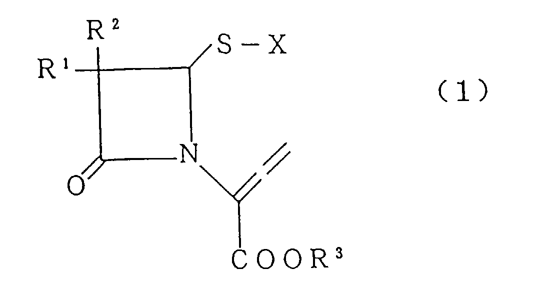 dans laquelle R¹ est un atome d'hydrogène, un atome d'halogène, un groupe amino ou amino protégé, R² est un atome d'hydrogène, un atome d'halogène, un groupe alcoxy inférieur en C₁-C₄, un groupe acyle inférieur en C₁-C₄, un groupe alkyle inférieur en C₁-C₄ substitué par un groupe hydroxyle ou hydroxyle protégé, un groupe hydroxyle ou hydroxyle protégé, R¹ et R² représentant =O lorsqu'ils sont pris ensemble, R³ est un atome d'hydrogène ou un groupe protecteur d'acide carboxylique, et X est le groupe -SO₂R⁴ ou le groupe -SR⁴, R⁴ étant un groupe aryle substitué ou non substitué ou un groupe hétérocyclique aromatique azoté substitué ou non substitué, le ou les substituants de R⁴ étant choisis parmi les halogènes, les groupes hydroxyle, nitro, cyano, aryle, alkyle inférieurs en C₁-C₄, amino, mono(alkyle inférieur en C₁-C₄)amino, di(alkyle inférieur en C₁-C₄)amino, mercapto, alkylthio ou arylthio représentés par le groupe R⁶S- (où R⁶ est un groupe alkyle inférieur en C₁-C₄ ou aryle), formyloxy, acyloxy représentés par le groupe R⁶COO-, formyle, acyle représentés par le groupe R⁶CO-, alcoxy ou aryloxy représentés par le groupe R⁶O-, carboxyle, alcoxycarbonyle ou aryloxycarbonyle représentés par le groupe R⁶OCO-.

2. Un procédé de préparation d'un allényl-β-lactame de formule (1) selon la revendication 1, caractérisé en ce qu'on fait réagir un dérivé d'azétidone représenté par la formule (2) 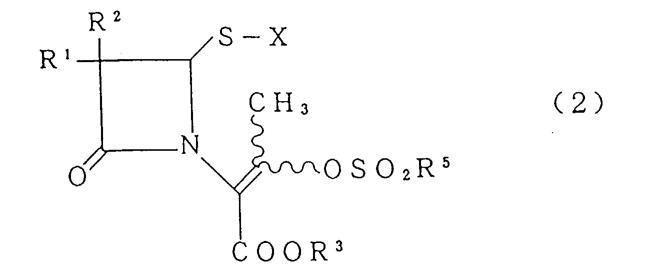 où R¹, R², R³ et X sont tels que définis dans la revendication 1 et R⁵ est un groupe alkyle inférieur en C₁-C₄ substitué ou non substitué ou un groupe aryle substitué ou non substitué, le ou les substituants étant définis comme pour R⁴ dans la revendication 1, avec une base.

3. L'utilisation d'un allényl-β-lactame de formule (1) selon la revendication 1 comme composé intermédiaire dans la synthèse d'un antibiotique du type péname, pénème ou céphème.
